# EUROPEAN PATENT SPECIFICATION

(11) **EP 3 226 830 B1**
(45) Date of publication and mention of the grant of the patent: **09.08.2023**
(21) Application number: 14806524.6
(22) Date of filing: 31.10.2014
(51) Int. Cl.: A61K 8/34, A61K 8/39, A61Q 15/00, A61K 8/86, A61K 8/891, A61K 8/92, A61K 8/02, A61K 8/06

(54) **ANTIPERSPIRANT COMPOSITION COMPRISING LIQUID CRYSTALS**
SCHWEISSHEMMENDE ZUSAMMENSETZUNG MIT FLÜSSIGKRISTALLEN
COMPOSITION ANTI-TRANSPIRANTE COMPRENANT DES CRISTAUX LIQUIDES

(43) Date of publication of application: 11.10.2017
(73) Proprietor: L'OREAL, 75008 Paris (FR)
(72) Inventor: MIRANDA, Amanda Rezende, 21535-501 Rio de Janeiro/RJ (BR); BARROSO, Pedro Tupinamba Werneck, 21535-501 Rio de Janeiro/RJ (BR); GAUDRY, Anne-Laure Carmen Paulette, 21535-501 Rio de Janeiro/RJ (BR)
(74) Representative: Lavoix
(86) International application number: PCT/BR2014/000401
(87) International publication number: WO 2016/065440

(56) References cited:
- WO-A1-02/083091
- WO-A1-2010/145909
- WO-A2-2013/087516
- US-A- 5 672 340
- US-A1- 2006 029 624

## Description

### FIELD OF THE INVENTION

The present invention relates to a method and composition for controlling body odor and perspiration from localized areas of the body such as the underarm.

### BACKGROUND OF THE INVENTION

Sweating, though a natural bodily function, results in the formation of wet patches on human skin or clothing. Moreover, the sweat secreted from sweat glands has a tendency to form a malodorous odor due to its interaction with the many microorganisms which reside on the underarm skin surface. These two phenomena are deemed to be undesirable in many societies.

Products are commonly available which help to address both phenomena. More particularly, antiperspirant products possess active ingredients which inhibit/suppress sweating, whereas deodorant products possess ingredients that mask the malodors caused by sweat. Many products address both phenomena simultaneously. An antiperspirant product is for example described in WO 2013/087516 A2.

In general, the above-referenced products fall into three dispensing category types: sprays, solids and roll-ons. Roll-on dispensers contain compositions comprised of liquids with low viscosity dispensed via a roller that sits loosely within a housing whereby the roller rotates across both the skin depositing the composition from the open end of the housing, and the fluid reservoir where it picks up the composition from the closed end of the housing.

The liquid compositions used in roll-on products possess several drawbacks. First, they have a tendency to impart an unpleasant sticky sensation onto skin when applied thereon. Secondly, they oftentimes take too long to dry after application. Lastly, the tactile properties associated with the product as its being applied, i.e. the way it "feels" during application, is typically unpleasant to end users.

It is thus an object of the present invention to provide an antiperspirant plus deodorant composition which avoids the above-referenced drawbacks.

### BRIEF SUMMARY OF THE INVENTION

The present invention relates to a cosmetic composition comprising:
(a) at least one antiperspirant active;
(b) an oil system comprising:
   (i) at least one plant derived oil; and
   (ii) at least one non-volatile silicone oil, as defined below;
(c) at least one wax, the wax being a fatty alcohol;
(d) an emulsifier system comprised of:
   (i) at least one high HLB emulsifier; and
   (ii) at least one low HLB emulsifier; and
(e) water,
wherein (b) and (d) are employed in a ratio by weight of about 10:1 to 2:1, the composition has a weighted average HLB of about 4 to 15, and the composition has liquid crystals present therein. Thereby it is understood, that the high HLB emulsifier has a HLB value in the range from 10 to 19 and the low HLB emulsifier has a HLB value in the range from 3 to 8.

The present invention is also directed to a process for making a cosmetic composition involving the steps of:
(a) providing at least one antiperspirant active;
(b) providing an oil system comprising:
   (i) at least one plant derived oil; and
   (ii) at least one non-volatile silicone oil, as defined below;
(c) providing at least one wax, the wax being a fatty alcohol;
(d) providing an emulsifier system comprised of:
   (i) at least one high HLB emulsifier; and
   (ii) at least one low HLB emulsifier;
(e) providing water; and
(f) combining (a)-(f) to form the composition,
wherein (b) and (d) are employed in a ratio by weight of about 10:1 to 2:1, the composition has a weighted average HLB of about 4 to 15, and the composition has liquid crystals present therein. Thereby it is understood, that the high HLB emulsifier has a HLB value in the range from 10 to 19 and the low HLB emulsifier has a HLB value in the range from 3 to 8.

The present invention is also directed to a process for suppressing sweat and odor from forming on human skin involving applying the above-disclosed composition onto a surface of the human skin.

Applicants have unexpectedly discovered that the above-described oil-in-water emulsion compositions exhibit faster drying times, possess greatly reduced stickiness and impart a moisturizing sensation upon application which is positively perceived by the end user, as compared to conventional oil-in-water emulsion roll-on products such as those of the prior art.

### DETAILED DESCRIPTION OF THE INVENTION

As used herein, the expression "at least one" means one or more and thus includes individual components as well as mixtures/combinations.

"Cosmetically acceptable" means that the item in question is compatible with any keratinous substrate. For example, "cosmetically acceptable carrier" means a carrier that is compatible with any keratinous substrate.

### ANTIPERSPIRANT ACTIVE

The term "antiperspirant active" is understood to mean any substance capable of reducing the flow of sweat. The antiperspirant active which can be used according to the invention are preferably chosen from aluminium and/or zirconium salts; complexes of zirconium chlorohydrate and of aluminium chlorohydrate with an amino acid.

Mention may in particular be made, among the aluminium salts, of aluminium chlorohydrate in the activated or nonactivated form, aluminium chlorohydrex, the aluminium chlorohydrex polyethylene glycol complex, the aluminium chlorohydrex propylene glycol complex, aluminium dichlorohydrate, the aluminium dichlorohydrex polyethylene glycol complex, the aluminium dichlorohydrex propylene glycol complex, aluminium sesqui-chlorohydrate, the aluminium sesquichlorohydrex polyethylene glycol complex, the aluminium sesqui¬chlorohydrex propylene glycol complex, or aluminium sulphate buffered by sodium aluminium lactate.

Mention may in particular be made, among the aluminium and zirconium salts, of aluminium zirconium octachlorohydrate, aluminium zirconium pentachloro-hydrate, aluminium zirconium tetrachlorohydrate or aluminium zirconium trichlorohydrate.

The complexes of zirconium chlorohydrate and of aluminium chlorohydrate with an amino acid are generally known under the name ZAG (when the amino acid is glycine). They are disclosed in particular in Patent US-3 792 068. The ZAG complexes usually exhibit an Al/Zr quotient ranging from approximately 1.67 to 12.5 and a Metal/Cl quotient ranging from approximately 0.73 to 1.93. Mention may be made, among these products, of the aluminium zirconium octachlorohydrex glycine, aluminium zirconium penta-chlorohydrex glycine, aluminium zirconium tetrachloro-hydrex glycine and aluminium zirconium trichlorohydrex glycine complexes.

Use will more particularly be made of aluminium chlorohydrate in activated or nonactivated form, in particular the product sold by SummitReheis under the name CHLORHYDROL^{®} 50 or LOCRON^{®} L.ZA by Clariant.

The antiperspirant actives are preferably present in the composition according to the invention in an amount of from 0.001 to 40% by weight, and preferably in an amount of from 0.1 to 25% by weight, based on the total weight of the composition.

### OIL SYSTEM

The composition of the present invention contains an oil system comprised of at least one plant derived oil and at least one non-volatile silicone oil, as defined below.

### PLANT-DERIVED OIL

The oil system of the present invention contains at least one plant-derived oil.

Plant-derived oils are those typically extracted from a plant's seeds, although equivalents which can be synthetically manufactured may be employed as well. Suitable plant derived oils include, but are not limited to, soybean oil, jojoba oil, coconut oil, safflower oil, palm kernel oil, cottonseed oil, and pine nut oil.

The plant-derived oils are typically present in the composition according to the invention in an amount of from 0.1 to 10%, such as from 1 to 6%, preferably from 3 to 4% by weight, based on the total weight of the composition.

### NON-VOLATILE SILICONE OIL

The oil system also contains at least one non-volatile silicone oil. Non-volatile silicone oils are defined in a known way as compounds which are non-volatile at ambient temperature (20-25°C)

Non-volatile silicone oils are defined in a known way as compounds with a low vapor pressure at ambient temperature. The nonvolatile silicone oils of the present invention are nonvolatile polydimethylsiloxanes (PDMS), polydimethylsiloxanes comprising alkyl or alkoxy groups that are pendent and/or at the end of a silicone chain, these groups each containing from 2 to 24 carbon atoms, phenyl silicones, for instance phenyl trimethicones, phenyl dimethicones, phenyl trimethylsiloxy diphenylsiloxanes, diphenyl dimethicones, diphenyl methyldiphenyl trisiloxanes and 2-phenylethyl trimethylsiloxysilicates, and dimethicones or phenyltrimethicones with a viscosity range of 0.25 to 3.5 cm²/s (25 to 350 cSt). A commercially available dimethicone having a viscosity of 1 cm²/s (100cSt) is sold under the trade name BELSIL^{®} DM 100 by Wacker.

The non-volatile silicone oils are typically present in the composition according to the invention in an amount of from 0.1 to 10%, such as from 1 to 6%, and preferably from 2 to 3% by weight, based on the total weight of the composition.

### WAX

The fatty phase comprises one or more waxes. A wax is defined as a fatty compound substantially in the form of a solid at ambient temperature (20-25°C) under atmospheric pressure (760 mmHg), and has a melting point generally of 35°C or more.

According to the present invention, the wax is a fatty alcohol. A fatty alcohol is defined as any saturated or unsaturated, linear or branched C8-C30 fatty alcohol, which is optionally substituted, in particular with one or more hydroxyl groups (in particular 1 to 4). If they are unsaturated, these compounds may comprise one to three conjugated or nonconjugated carbon-carbon double bonds. Preferably fatty alcohols are unsaturated and/or branched. Among the C8-C30 fatty alcohols, C12-C22 fatty alcohols, for example, are used. Mention may be made among these of isostearyl alcohol, oleyl alcohol, linoleyl alcohol, undecylenyl alcohol, palmitoleyl alcohol, linolenyl alcohol, erucyl alcohol, and mixtures thereof. In one embodiment, cetyl alcohol, stearyl alcohol or a mixture thereof (e.g., cetearyl alcohol), as well as myristyl alcohol, can be used as a solid fatty material.

The wax is present in the composition according to the invention in an amount of from 0,1 to 4% by weight, such as from 0.5 to 3% by weight, and preferably from 1.5 to 2.5% by weight, all weights based on the total weight of the composition.

### EMULSIFIER SYSTEM

An essential constituent of the oil-in-water emulsions of the present invention is a mixture of non-ionic emulsifiers forming an emulsifier system. Such an emulsifier system conveniently has a weighted average HLB value in the region of from about 4 to about 15 and particularly from 6 to 9. An especially desired weighted average HLB value is from 6 to 6.29.

Such a weighted average HLB value is obtained by using a combination of at least two emulsifiers, a first (lower) HLB emulsifier having an HLB value in the range of from 3 to 8, and in particular from 4 to 6 and a second (higher) HLB emulsifier having an HLB value in the range of from 10 to 19 and especially from 14 to 16. When a combination of emulsifiers is employed, the weighted average HLB value is obtained by varying the weight average of the low and high HLB values of the constituent emulsifiers. The weighted average HLB value is obtained by calculating the weighted average of the individual HLB values.

The non-ionic emulsifiers may be chosen from ethoxylated non-ionic surfactants and propoxylated non-ionic surfactants. The combination of emulsifiers includes, but is not limited to, steareth-2 (HLB = 4.9) and one selected from steareth-15 (HLB = 14.2) to steareth-100 (HLB = 18.8). Particularly preferred is a combination of steareth-2 and steareth-20.

It is desirable to use a mixture of emulsifiers in a weight ratio of emulsifier having a lower HLB value of <6.5 to emulsifier having a higher HLB value of >8 of from about 1.5:1 to 6:1 and particularly from 2:1 to 5:1.

The emulsifiers are present in the composition according to the invention in an amount of from 0.1 to 3% by weight, such as from 0.1 to 1.5% by weight, and from 0.2 to 1.3% by weight, all weights based on the total weight of the composition.

One of the surprising properties observed by the composition of the present invention is its ability to impart a pleasant feeling of moisturizing which is perceived by the end user upon application to their skin. This tactile feeling is directly related to liquid crystals which are formed in the composition based on the selection and association of the constituents of the oil system and emulsifier system.

More particularly, it is imperative that the ratio by weight of the oil system to the emulsifier system be in the range of 10:1 to 2:1, such as 7:1 to 2:1, and 3:1 to 4.7:1. It has been found that by using the above-disclosed oil and emulsifier systems, in the disclosed amounts, liquid crystals are formed within the composition which yield the desired moisturizing tactile properties observed by the end user.

The aqueous phase of the said emulsions comprises water and generally other water-soluble or water-miscible solvents. The water-soluble or water-miscible solvents comprise short-chain, for example C1-C4, monoalcohols, such as ethanol or isopropanol; or diols or polyols, such as ethylene glycol, 1,2-propylene glycol, 1,3-butylene glycol, hexylene glycol, diethylene glycol, dipropylene glycol, 2-ethoxyethanol, diethylene glycol monomethyl ether, triethylene glycol monomethyl ether and sorbitol.

The compositions according to the invention may additionally comprise moisture or odor absorbers, for instance perlites and preferably expanded perlites such as the material sold under the trade name OPTIMAT^{®} 2550 OR by World Minerals.

The cosmetic compositions according to the invention may additionally comprise cosmetic adjuvants chosen from fragrances, chelating agents, softeners, antioxidants, opacifiers, stabilizers, moisturizing agents, vitamins, bactericides, preservatives, polymers, thickening agents, or any other ingredient commonly used in cosmetics for this type of application.

Of course, a person skilled in the art will take care to choose this or these optional additional compounds so that the advantageous properties intrinsically attached to the cosmetic composition in accordance with the invention are not, or not substantially, detrimentally affected by the envisaged addition or additions.

The amounts of these various constituents which can be present in the cosmetic composition according to the invention are those conventionally used in antiperspirant roll-ons, twist sticks and creams.

The antiperspirant cosmetic composition according to the invention can be provided in the more or less thickened form distributed in a tube or in a twist stick or in the form of a roll-on packaged in the ball form and can, in this respect, comprise the ingredients generally used in products of this type and well known to a person skilled in the art.

The following examples serve to illustrate the invention. In these examples the amounts of the composition ingredients are given as weight percentages relative to the total weight of the composition.

### EXAMPLES

**TABLE 1: INVENTIVE EXAMPLES**

| **INGREDIENTS (US INCI NAME)** | | **Example 1** | **Example 2** | **Example 3** (out of the invention) |
|---|---|---|---|---|
| WATER | | QS | QS | QS |
| Oil System: | | | | |
| | ELAEIS GUINEENSIS (PALM) OIL | 4.0 | 4.0 | 4.0 |
| | DIMETHICONE (100 cSt) | 3.0 | 3.0 | 3.0 |
| Emulsifier System: | | | | |
| | STEARETH-2 | 1.3 | 1.3 | 2.5 |
| | STEARETH-20 | 0.2 | 0.73 | 1.2 |
| CETEARYL ALCOHOL | | 2.5 | 2.5 | 2.5 |
| TETRASODIUM GLUTAMATE DIACETATE | | 0.08 | 0.08 | 0.08 |
| ALUMINUM CHLOROHYDRATE | | 15.0 | 15.0 | 15.0 |
| PERLITE | | 0 | 1.0 | 1.0 |
| FRAGRANCE | | 1.4 | 1.4 | 1.4 |
| PRESERVATIVE | | 0.5 | 0.5 | 0.5 |
| | TOTAL (%) | 100.0 | 100.0 | 100.0 |
| **Oil System (%)** | | **7.0** | **7.0** | **7.0** |
| **Emulsifier System (%)** | | **1.5** | **2.03** | **3.7** |
| **Ratio Oil System : Emulsifier System** | | **4.7:1** | **3.5:1** | **1.9:1** |
| **Weighted Average HLB of Emulsifiers** | | **6.29** | **8.6** | **8.3** |
| **Observation: Liquid Crystals** | | **Yes** | **Yes** | **Yes** |

**TABLE 2: COMPARATIVE EXAMPLES**

| **INGREDIENTS (US INCI NAME)** | | **Example 4** |
|---|---|---|
| WATER | | as |
| Oil System: | | |
| | DIMETHICONE (350 cSt) | 0.5 |
| Emulsifier System: | | |
| | CETEARETH-33 | 1.25 |
| CETEARYL ALCOHOL | | 2.5 |
| ALUMINUM CHLOROHYDRATE | | 10 |
| PERLITE | | 1.0 |
| TALC | | 1.0 |
| FRAGRANCE | | 1.0 |
| PRESERVATIVE | | 0.5 |
| PRESERVATIVE | | 0.1 |
| TOTAL (%) | | 100.0 |
| **Oil System (%)** | | **0.5** |
| **Emulsifier System (%)** | | **1.25** |
| **Ratio Oil System : Emulsifier System** | | **1:2.5** |
| **Weighted Average HLB of Emulsifiers** | | **16.8** |
| **Observation: Liquid Crystals** | | **No** |

In making each of the examples in Tables 1, the following procedure was used. In the annex, elaeis guineensis (palm oil), dimethicone, cetearyl alcohol, steareth-2 and steareth-20 were combined with heating to 65°C. The contents of the annex were added to the main vessel containing water at 65°C. The contents of the main vessel were emulsified then cooled to 40°C. Tetrasodium glutamate diacetate was introduced to the main vessel followed by the slow introduction of aluminum chlorohydrate and water, and the contents were homogenized. Perlite, fragrance, and preservatives were added, and the contents of the main vessel were homogenized.

Samples were examined for liquid crystals by optical microscopy (200x magnification) under polarized light.

**TABLE 3: DRYING TIME COMPARISON**

| **Test Product** | **Number of Turns with Fingertip** |
|---|---|
| Example 1 | 116.409 |
| Dove Original com Vitamina E e F | 148.773 |
| Antitranspiante Roll-on 48h | |

Drying time was determined by a trained panel comprised of 14 panelists who assessed the properties of the formulas on their forearm. Each panelist assessed the drying time of the product by counting the number of turns with her fingertips until the product was considered to feel dry. A higher number indicates that the product took longer to feel dry.

**TABLE 4: SENSORIAL TACKINESS COMPARISON**

| **Test Product** | **Value (0-10)** |
|---|---|
| Example 1 | 1.736 |
| Dove Original con Vitamina E e F Antitranspiante Roll-on 48h | 5.005 |

Tackiness was determined by a trained panel comprised of 14 panelists who assessed the skin feel properties of the formulas on their forearm. Each panelist assessed the tackiness of the product by feeling the product with her fingertips and rating the tackiness on a scale of 0 (no tack) to 10 (very tacky).

## Claims

1. A method of making a composition for use as an antiperspirant and deodorant product comprising:
(a) providing antiperspirant active;
(b) providing an oil system comprising:
(i) at least one plant derived oil; and
(ii) at least one non-volatile silicone oil which is non-volatile at a temperature comprised between 20°C and 25°C, said non-volatile silicone oil being chosen from the group consisting of: nonvolatile polydimethylsiloxanes (PDMS), polydimethylsiloxanes comprising alkyl or alkoxy groups that are pendent and/or at the end of a silicone chain, these groups each containing from 2 to 24 carbon atoms, phenyl silicones, and dimethicones or phenyltrimethicones with a viscosity range of 25 to 350 cSt;
(c) providing at least one wax which is a fatty alcohol;
(d) providing an emulsifier system comprised of:
(i) at least one high HLB emulsifier having an HLB value in the range of from 10 to 19; and
(ii) at least one low HLB emulsifier having an HLB value in the range of from 3 to 8;
(e) providing water; and
(f) combining (a)-(f) to form the composition,
wherein (b) and (d) are employed in a ratio by weight of 10:1 to 2:1, wherein the composition has a weighted average HLB of from 4 to 15, and wherein the composition has liquid crystals present therein.

2. The method of claim 1 wherein (b)(i) is employed in an amount of from 0.1 to 10% by weight, based on the weight of the composition.

3. The method of claim 1 wherein (b)(i) is palm oil.

4. The method of claim 1 wherein (b)(ii) is employed in an amount of from 0.1 to 10% by weight, based on the weight of the composition.

5. The method of claim 1 wherein (b)(ii) is dimethicone.

6. The method of claim 1 wherein (c) is employed in an amount of from 0.1 to 4% by weight, based on the weight of the composition.

7. The method of claim 1 wherein (c) is cetearyl alcohol.

8. The method of claim 1 wherein (d)(i) is employed in an amount of from 0.1 to 3% by weight, based on the weight of the composition.

9. The method of claim 1 wherein (d)(i) is steareth-20.

10. The method of claim 1 wherein (d)(ii) is employed in an amount of from 0.1 to 1.5% by weight, based on the weight of the composition.

11. The method of claim 1 wherein (d)(ii) is steareth-2.

12. A method of making a composition for use as an antiperspirant and deodorant product comprising:
(a) providing from 10 to 15% by weight of an antiperspirant active;
(b) providing an oil system comprising:
(i) from 3 to 4% by weight of palm; and
(ii) from 2 to 3 % by weight of dimethicone;
(c) providing from 1.5 to 2.5% by weight of cetearyl alcohol;
(d) providing an emulsifier system comprised of:
(i) from 0.1 to 0.5 % by weight of steareth-20; and
(ii) from 1 to 1.3 % by weight of steareth-2;
(e) providing remainder, to 100%, water, all weights based on the total weight of the composition; and
(f) combining (a)-(f) to form the composition,
wherein (b) and (d) are employed in a ratio by weight of 4.7:1, wherein the composition has a weighted average HLB of from 6 to 6.29, and wherein the composition has liquid crystals present therein.

13. A composition for use as an antiperspirant and deodorant product comprising:
(a) providing antiperspirant active;
(b) providing an oil system comprising:
(i) at least one plant derived oil; and
(ii) at least one non-volatile silicone oil which is non-volatile at a temperature comprised between 20°C and 25°C, said non-volatile silicone oil being chosen from the group consisting of: nonvolatile polydimethylsiloxanes (PDMS), polydimethylsiloxanes comprising alkyl or alkoxy groups that are pendent and/or at the end of a silicone chain, these groups each containing from 2 to 24 carbon atoms, phenyl silicones, and dimethicones or phenyltrimethicones with a viscosity range of 25 to 350 cSt;
(c) providing at least one wax which is a fatty alcohol;
(d) providing an emulsifier system comprised of:
(i) at least one high HLB emulsifier having an HLB value in the range of from 10 to 19; and
(ii) at least one low HLB emulsifier having an HLB value in the range of from 3 to 8;
(e) providing water; and
(f) combining (a)-(f) to form the composition,
wherein (b) and (d) are employed in a ratio by weight of 10:1 to 2:1, wherein the composition has a weighted average HLB of from 4 to 15, and wherein the composition has liquid crystals present therein.

14. The composition of claim 13 wherein (b)(i) is employed in an amount of from 0.1 to 10% by weight, based on the weight of the composition.

15. The composition of claim 13 wherein (b)(i) is palm oil.

16. The composition of claim 13 wherein (b)(ii) is employed in an amount of from 0.1 to 10% by weight, based on the weight of the composition.

17. The composition of claim 13 wherein (b)(ii) is dimethicone.

18. The composition of claim 13 wherein (c) is employed in an amount of from 0.1 to 4% by weight, based on the weight of the composition.

19. The composition of claim 13 wherein (c) is cetearyl alcohol.

20. The composition of claim 13 wherein (d)(i) is employed in an amount of from 0.1 to 3% by weight, based on the weight of the composition.

21. The composition of claim 13 wherein (d)(i) is steareth-20.

22. The composition of claim 13 wherein (d)(ii) is employed in an amount of from 0.1 to 1.5% by weight, based on the weight of the composition.

23. The composition of claim 13 wherein (d)(ii) is steareth-2.

24. A composition for use as an antiperspirant and deodorant product comprising:
(a) providing from 10 to 15% by weight of an antiperspirant active;
(b) providing an oil system comprising:
(i) from 3 to 4% by weight of palm; and
(ii) from 2 to 3% by weight of dimethicone;
(c) providing from 1.5 to 2.5% by weight of cetearyl alcohol;
(d) providing an emulsifier system comprised of:
(i) from 0.1 to 0.5% by weight of steareth-20; and
(ii) from 1 to 1.3% by weight of steareth-2;
(e) providing remainder, to 100%, water, all weights based on the total weight of the composition; and
(f) combining (a)-(f) to form the composition,
wherein (b) and (d) are employed in a ratio by weight of 4.7:1, wherein the composition has a weighted average HLB of from 6 to 6.29, and wherein the composition has liquid crystals present therein.

## Patentansprüche

1. Verfahren zum Herstellen einer Zusammensetzung zur Verwendung als schweißhemmendes und deodorierendes Produkt, umfassend:
(a) Bereitstellen eines schweißhemmenden Wirkstoffs;
(b) Bereitstellen eines Ölsystems, umfassend:
(i) mindestens ein von Pflanzen abgeleitetes Öl; und
(ii) mindestens ein nicht flüchtiges Silikonöl, das bei einer Temperatur zwischen 20 °C und 25 °C nicht flüchtig ist, wobei das nicht flüchtige Silikonöl ausgewählt ist aus der Gruppe, bestehend aus: nicht flüchtigen Polydimethylsiloxanen (PDMS), Polydimethylsiloxanen, umfassend seitenständige und/oder am Ende einer Silikonkette befindliche Alkyl- oder Alkoxygruppen, wobei diese Gruppen jeweils 2 bis 24 Kohlenstoffatome enthalten, Phenylsiliconen, Dimethiconen oder Phenyltrimethiconen mit einer Viskosität in einem Bereich zwischen 25 und 350 cSt;
(c) Bereitstellen mindestens eines Wachses, das ein Fettalkohol ist;
(d) Bereitstellen eines Emulgatorsystems, bestehend aus:
(i) mindestens einem Hoch-HLB-Emulgator, der einen HLB-Wert in dem Bereich von 10 bis 19 aufweist; und
(i) mindestens einem Niedrig-HLB-Emulgator, der einen HLB-Wert in dem Bereich von 3 bis 8 aufweist;
(e) Bereitstellen von Wasser; und
(f) Kombinieren von (a)-(f), um die Zusammensetzung zu bilden,
wobei (b) und (d) in einem Gewichtsverhältnis von 10:1 bis 2:1 eingesetzt werden, wobei die Zusammensetzung einen gewichteten durchschnittlichen HLB-Wert von 4 bis 15 aufweist und wobei die Zusammensetzung Flüssigkristalle darin aufweist.

2. Verfahren nach Anspruch 1, wobei (b)(i) in einer Menge von 0,1 bis 10 Gewichtsprozent, bezogen auf das Gewicht der Zusammensetzung, eingesetzt wird.

3. Verfahren nach Anspruch 1, wobei (b)(i) Palmöl ist.

4. Verfahren nach Anspruch 1, wobei (b)(ii) in einer Menge von 0,1 bis 10 Gewichtsprozent, bezogen auf das Gewicht der Zusammensetzung, eingesetzt wird.

5. Verfahren nach Anspruch 1, wobei (b)(ii)) Dimethicon ist.

6. Verfahren nach Anspruch 1, wobei (c) in einer Menge von 0,1 bis 4 Gewichtsprozent, bezogen auf das Gewicht der Zusammensetzung, eingesetzt wird.

7. Verfahren nach Anspruch 1, wobei (c) Cetearylalkohol ist.

8. Verfahren nach Anspruch 1, wobei (d)(i) in einer Menge von 0,1 bis 3 Gewichtsprozent, bezogen auf das Gewicht der Zusammensetzung, eingesetzt wird.

9. Verfahren nach Anspruch 1, wobei (d)(i) Steareth-20 ist.

10. Verfahren nach Anspruch 1, wobei (d)(ii) in einer Menge von 0,1 bis 1,5 Gewichtsprozent, bezogen auf das Gewicht der Zusammensetzung, eingesetzt wird.

11. Verfahren nach Anspruch 1, wobei (d)(ii) Steareth-2 ist.

12. Verfahren zum Herstellen einer Zusammensetzung zur Verwendung als schweißhemmendes und deodorierendes Produkt, umfassend:
(a) Bereitstellen von 10 bis 15 Gewichtsprozent eines schweißhemmenden Wirkstoffs;
(b) Bereitstellen eines Ölsystems, umfassend:
(i) 3 bis 4 Gewichtsprozent Palmöl; und
(ii) 2 bis 3 Gewichtsprozent Dimethicon;
(c) Bereitstellen von 1,5 bis 2,5 Gewichtsprozent Cetearylalkohol;
(d) Bereitstellen eines Emulgatorsystems, bestehend aus:
(i) 0,1 bis 0,5 Gewichtsprozent Steareth-20; und
(ii) 1 bis 1,3 Gewichtsprozent Steareth-2;
(e) Bereitstellen eines Rests von 100 % Wasser, wobei alle Gewichte auf das Gesamtgewicht der Zusammensetzung bezogen sind; und
(f) Kombinieren von (a)-(f), um die Zusammensetzung zu bilden,
wobei (b) und (d) in einem Gewichtsverhältnis von 4,7:1 eingesetzt werden, wobei die Zusammensetzung einen gewichteten durchschnittlichen HLB-Wert von 6 bis 6,29 aufweist und wobei die Zusammensetzung Flüssigkristalle darin aufweist.

13. Zusammensetzung zur Verwendung als schweißhemmendes und deodorierendes Produkt, umfassend:
(a) Bereitstellen eines schweißhemmenden Wirkstoffs;
(b) Bereitstellen eines Ölsystems, umfassend:
(i) mindestens ein von Pflanzen abgeleitetes Öl; und
(ii) mindestens ein nicht flüchtiges Silikonöl, das bei einer Temperatur zwischen 20 °C und 25 °C nicht flüchtig ist, wobei das nicht flüchtige Silikonöl ausgewählt ist aus der Gruppe, bestehend aus: nicht flüchtigen Polydimethylsiloxanen (PDMS), Polydimethylsiloxanen, umfassend seitenständige und/oder am Ende einer Silikonkette befindliche Alkyl- oder Alkoxygruppen, wobei diese Gruppen jeweils 2 bis 24 Kohlenstoffatome enthalten, Phenylsiliconen, Dimethiconen oder Phenyltrimethiconen mit einer Viskosität in einem Bereich zwischen 25 und 350 cSt;
(c) Bereitstellen mindestens eines Wachses, das ein Fettalkohol ist;
(d) Bereitstellen eines Emulgatorsystems, bestehend aus:
(i) mindestens einem Hoch-HLB-Emulgator, der einen HLB-Wert in dem Bereich von 10 bis 19 aufweist; und
(i) mindestens einem Niedrig-HLB-Emulgator, der einen HLB-Wert in dem Bereich von 3 bis 8 aufweist;
(e) Bereitstellen von Wasser; und
(f) Kombinieren von (a)-(f), um die Zusammensetzung zu bilden,
wobei (b) und (d) in einem Gewichtsverhältnis von 10:1 bis 2:1 eingesetzt werden, wobei die Zusammensetzung einen gewichteten durchschnittlichen HLB-Wert von 4 bis 15 aufweist und wobei die Zusammensetzung Flüssigkristalle darin aufweist.

14. Zusammensetzung nach Anspruch 13, wobei (b)(i) in einer Menge von 0,1 bis 10 Gewichtsprozent, bezogen auf das Gewicht der Zusammensetzung, eingesetzt wird.

15. Zusammensetzung nach Anspruch 13, wobei (b)(i) Palmöl ist.

16. Zusammensetzung nach Anspruch 13, wobei (b)(ii) in einer Menge von 0,1 bis 10 Gewichtsprozent, bezogen auf das Gewicht der Zusammensetzung, eingesetzt wird.

17. Zusammensetzung nach Anspruch 13, wobei (b)(ii) Dimethicon ist.

18. Zusammensetzung nach Anspruch 13, wobei (c) in einer Menge von 0,1 bis 4 Gewichtsprozent, bezogen auf das Gewicht der Zusammensetzung, eingesetzt wird.

19. Zusammensetzung nach Anspruch 13, wobei (c) Cetearylalkohol ist.

20. Zusammensetzung nach Anspruch 13, wobei (d)(i) in einer Menge von 0,1 bis 3 Gewichtsprozent, bezogen auf das Gewicht der Zusammensetzung, eingesetzt wird.

21. Zusammensetzung nach Anspruch 13, wobei (d)(i) Steareth-20 ist.

22. Zusammensetzung nach Anspruch 13, wobei (d)(ii) in einer Menge von 0,1 bis 1,5 Gewichtsprozent, bezogen auf das Gewicht der Zusammensetzung, eingesetzt wird.

23. Zusammensetzung nach Anspruch 13, wobei (d)(ii) Steareth-2 ist.

24. Zusammensetzung zur Verwendung als schweißhemmendes und deodorierendes Produkt, umfassend:
(a) Bereitstellen von 10 bis 15 Gewichtsprozent eines schweißhemmenden Wirkstoffs;
(b) Bereitstellen eines Ölsystems, umfassend:
(i) 3 bis 4 Gewichtsprozent Palmöl; und
(ii) 2 bis 3 Gewichtsprozent Dimethicon;
(c) Bereitstellen von 1,5 bis 2,5 Gewichtsprozent Cetearylalkohol;
(d) Bereitstellen eines Emulgatorsystems, bestehend aus:
(i) 0,1 bis 0,5 Gewichtsprozent Steareth-20; und
(ii) 1 bis 1,3 Gewichtsprozent Steareth-2;
(e) Bereitstellen eines Rests von 100 % Wasser, wobei alle Gewichte auf das Gesamtgewicht der Zusammensetzung bezogen sind; und
(f) Kombinieren von (a)-(f), um die Zusammensetzung zu bilden, wobei (b) und (d) in einem Gewichtsverhältnis von 4,7:1 eingesetzt werden, wobei die Zusammensetzung einen gewichteten durchschnittlichen HLB-Wert von 6 bis 6,29 aufweist und wobei die Zusammensetzung Flüssigkristalle darin aufweist.

## Revendications

1. Procédé de fabrication d'une composition destinée à être utilisée comme produit anti-transpirant et déodorant, comprenant :
(a) la fourniture d'un agent anti-transpirant ;
(b) la fourniture d'un système d'huile comprenant :
(i) au moins une huile d'origine végétale ; et
(ii) au moins une huile de silicone non volatile qui est non volatile à une température comprise entre 20 °C et 25 °C, ladite huile de silicone non volatile étant choisie dans le groupe constitué par : les polydiméthylsiloxanes (PDMS) non volatils, les polydiméthylsiloxanes comprenant des groupes alkyle ou alcoxy pendants et/ou à l'extrémité d'une chaîne de silicone, ces groupes contenant chacun de 2 à 24 atomes de carbone, les phényl silicones, et les diméthicones ou phényltriméthicones ayant une viscosité dans la plage de 25 à 350 cSt ;
(c) la fourniture d'au moins une cire qui est un alcool gras ;
(d) la fourniture d'un système émulsifiant composé de :
(i) au moins un émulsifiant à haut HLB ayant une valeur HLB dans la plage de 10 à 19; et
(ii) au moins un émulsifiant à faible HLB ayant une valeur HLB dans la plage de 3 à 8 ;
(e) la fourniture d'eau ; et
(f) la combinaison des points (a) à (f) pour former la composition,
dans lequel (b) et (d) sont employés dans un rapport en poids de 10:1 à 2:1, dans lequel la composition a un HLB moyen pondéré de 4 à 15, et dans lequel la composition contient des cristaux liquides.

2. Procédé selon la revendication 1 dans lequel (b)(i) est employé dans une quantité de 0,1 à 10 % en poids, par sur la base du poids de la composition.

3. Procédé selon la revendication 1, dans lequel (b)(i) est de l'huile de palme.

4. Procédé selon la revendication 1 dans lequel (b)(ii) est employé dans une quantité de 0,1 à 10 % en poids, sur la base du poids de la composition.

5. Procédé selon la revendication 1, dans lequel (b)(ii) est la diméthicone.

6. Procédé selon la revendication 1 dans lequel (c) est employé dans une quantité de 0,1 à 4 % en poids, sur la base du poids de la composition.

7. Procédé selon la revendication 1 dans lequel (c) est l'alcool cétéarylique.

8. Procédé selon la revendication 1 dans lequel (d)(i) est employé dans une quantité de 0,1 à 3 % en poids, sur la base du poids de la composition.

9. Procédé selon la revendication 1, dans lequel (d)(i) est le stéaréth-20.

10. Procédé selon la revendication 1 dans lequel (d)(ii) est employé en une quantité de 0,1 à 1,5 % en poids, sur la base du poids de la composition.

11. Procédé selon la revendication 1 dans lequel (d)(ii) est le stéaréth-2.

12. Procédé de fabrication d'une composition destinée à être utilisée comme produit anti-transpirant et déodorant, comprenant :
(a) la fourniture de 10 à 15 % en poids d'un actif anti-transpirant ;
(b) la fourniture d'un système d'huile comprenant :
(i) de 3 à 4 % en poids d'huile de palme ; et
(ii) de 2 à 3 % en poids de diméthicone ;
(c) la fourniture de 1,5 à 2,5 % en poids d'alcool cétéarylique ;
(d) la fourniture d'un système émulsifiant composé de :
(i) de 0,1 à 0,5 % en poids de stéaréth-20 ; et
(ii) de 1 à 1,3 % en poids de stéaréth-2 ;
(e) la fourniture du reste, jusqu'à 100 %, en eau, tous les poids étant fondés sur le poids total de la composition ; et
(f) la combinaison des points (a) à (f) pour former la composition,
dans lequel (b) et (d) sont employés dans un rapport en poids de 4,7:1, dans lequel la composition a un HLB moyen pondéré de 6 à 6,29, et dans lequel la composition contient des cristaux liquides.

13. Composition destinée à être utilisée comme produit anti-transpirant et déodorant comprenant :
(a) la fourniture d'un agent anti-transpirant ;
(b) la fourniture d'un système d'huile comprenant :
(i) au moins une huile d'origine végétale ; et
(ii) au moins une huile de silicone non volatile qui est non volatile à une température comprise entre 20 °C et 25 °C, ladite huile de silicone non volatile étant choisie dans le groupe constitué par : les polydiméthylsiloxanes (PDMS) non volatils, les polydiméthylsiloxanes comprenant des groupes alkyle ou alcoxy pendants et/ou à l'extrémité d'une chaîne de silicone, ces groupes contenant chacun de 2 à 24 atomes de carbone, les phényl silicones, et les diméthicones ou phényltriméthicones ayant une viscosité dans la plage de 25 à 350 cSt ;
(c) la fourniture d'au moins une cire qui est un alcool gras ;
(d) la fourniture d'un système émulsifiant composé de :
(i) au moins un émulsifiant à haut HLB ayant une valeur HLB dans la plage de 10 à 19; et
(ii) au moins un émulsifiant à faible HLB ayant une valeur HLB dans la plage de 3 à 8 ;
(e) la fourniture d'eau ; et
(f) la combinaison des points (a) à (f) pour former la composition,
dans lequel (b) et (d) sont employés dans un rapport en poids de 10:1 à 2:1, dans lequel la composition a un HLB moyen pondéré de 4 à 15, et dans lequel la composition contient des cristaux liquides.

14. Composition selon la revendication 13, dans laquelle (b)(i) est employé dans une quantité allant de 0,1 à 10 % en poids, sur la base du poids de la composition.

15. Composition selon la revendication 13 dans laquelle (b)(i) est de l'huile de palme.

16. Composition selon la revendication 13, dans laquelle (b)(ii) est employé dans une quantité allant de 0,1 à 10 % en poids, sur la base du poids de la composition.

17. Composition selon la revendication 13 dans laquelle (b)(ii) est une diméthicone.

18. Composition selon la revendication 13, dans laquelle (c) est employé dans une quantité de 0,1 à 4 % en poids, sur la base du poids de la composition.

19. Composition selon la revendication 13 dans laquelle (c) est l'alcool cétéarylique.

20. Composition selon la revendication 13, dans laquelle (d)(i) est employé dans une quantité de 0,1 à 3 % en poids, sur la base du poids de la composition.

21. Composition selon la revendication 13, dans laquelle (d)(i) est le stéaréth-20.

22. Composition selon la revendication 13 dans laquelle (d)(ii) est employé dans une quantité de 0,1 à 1,5 % en poids, sur la base du poids de la composition.

23. Composition selon la revendication 13, dans laquelle (d)(ii) est le stéaréth-2.

24. Composition destinée à être utilisée comme produit anti-transpirant et déodorant comprenant :
(a) la fourniture de 10 à 15 % en poids d'un actif anti-transpirant ;
(b) la fourniture d'un système d'huile comprenant :
(i) de 3 à 4 % en poids d'huile de palme ; et
(ii) de 2 à 3 % en poids de diméthicone ;
(c) la fourniture de 1,5 à 2,5 % en poids d'alcool cétéarylique ;
(d) la fourniture d'un système émulsifiant composé de :
(i) de 0,1 à 0,5 % en poids de stéaréth-20 ; et
(ii) de 1 à 1,3 % en poids de stéaréth-2 ;
(e) la fourniture du reste, jusqu'à 100 %, en eau, tous les poids étant fondés sur le poids total de la composition ; et
(f) la combinaison des points (a) à (f) pour former la composition, dans laquelle (b) et (d) sont employés dans un rapport en poids de 4,7:1, dans laquelle la composition a un HLB moyen pondéré de 6 à 6,29, et dans laquelle la composition contient des cristaux liquides.
